Europäisches Patentamt

European Patent Office

Office européen des brevets

(11) Veröffentlichungsnummer: **0 258 580 B1**

(12) **EUROPÄISCHE PATENTSCHRIFT**

(45) Veröffentlichungstag der Patentschrift: **04.03.92**

(21) Anmeldenummer: **87110053.3**

(22) Anmeldetag: **11.07.87**

Teilanmeldung 91100480.2 eingereicht am 11/07/87.

(51) Int. Cl.⁵: **A61M 5/14**, A61M 25/00

(54) **Implantierbare Kathetervorrichtung.**

(30) Priorität: **21.08.86 DE 3628337**

(43) Veröffentlichungstag der Anmeldung:
**09.03.88 Patentblatt 88/10**

(45) Bekanntmachung des Hinweises auf die
Patenterteilung:
**04.03.92 Patentblatt 92/10**

(84) Benannte Vertragsstaaten:
**AT BE CH DE ES FR GB GR IT LI NL SE**

(56) Entgegenhaltungen:
**DE-A- 2 735 086**
**DE-A- 2 831 267**
**DE-U- 8 434 177**
**FR-A- 2 493 149**

(73) Patentinhaber: **B. Braun Melsungen AG**
**Carl-Braun Strasse**
**W-3508 Melsungen(DE)**

(72) Erfinder: **Stöber, Herbert**
**Über dem Rötter 1**
**W-3513 Staufenberg 1(DE)**
Erfinder: **Brencher, Norbert**
**Danziger Strasse 33**
**W-3435 Hess. Lichtenau(DE)**

(74) Vertreter: **Selting, Günther, Dipl.-Ing. et al**
**Deichmannhaus am Hauptbahnhof**
**W-5000 Köln 1(DE)**

## Beschreibung

Die Erfindung betrifft eine implantierbare Kathetervorrichtung, insbesondere für die Langzeit-Zytostatika-Therapie.

Für gewisse Therapien ist eine ständige Zufuhr von Pharmazeutika in das Blutsystem eines Patienten erforderlich. Es ist bekannt, unter die Haut eines Patienten eine Kapsel zu implantieren, die mit einem Katheterschlauch verbunden ist, dessen anderes Ende in ein Gefäß hineinführt. Zum Einbringen eines Medikamentes wird eine Hohlnadel durch die Haut des Patienten hindurch in die Kapsel eingeführt. Die Kapsel weist zu diesem Zweck eine membranartige Oberwand auf, die mit der Hohlnadel durchstochen werden kann. Aus einer Spritze kann dann durch die Hohlnadel das Medikament in die Kapsel eingespritzt werden.

Bei einer bekannten implantierbaren Kathetervorrichtung (DE-B-33 09 788) weist die Kapsel einen die Bodenwand verlängernden, radial abstehenden Flansch auf. Oberhalb des Flansches befindet sich ein von der Umfangswand der Kapsel abstehender Anschlußstutzen, der weit über den Flansch hinausragt. Das Ende des Katheters ist mit einem flexiblen Schlauchstück versehen, das über das Anschlußstück gezogen werden kann, um den Katheter mit der Kapsel zu verbinden. Nachteilig ist hierbei, daß der Katheter eine vorgegebene Länge hat, die vom Anwender nicht gekürzt werden kann, weil anderenfalls das Schlauchstück, das auf den Anschlußstutzen aufgeschoben wird, mit abgeschnitten würde. Zur Ankopplung des Katheters an die Kapsel werden erhebliche Kräfte benötigt, um einen sicheren Sitz zu gewährleisten. Andererseits ist die Ankupplung des Katheters an den Anschlußstutzen nicht sicher, weil bei unsachgemäßer Handhabung der Katheter von dem Anschlußstutzen abgezogen werden kann. Schließlich ist nachteilig, daß der Anschlußstutzen weit über die Kontur der Kapsel hinaus vorsteht, so daß bei der Verlegung der Kapsel Probleme auftreten können.

Bei einer weiteren bekannten implantierbaren Kathetervorrichtung (DE-U-84 34 177) weist die Kapsel ebenfalls einen seitlich abstehenden Bodenflansch auf sowie einen den Bodenflansch seitlich überragenden Anschlußstutzen. Das Katheterende kann durch den Anschlußstutzen hindurch in den Hohlraum der Kapsel eingeführt werden und die Fixierung des Katheters am Anschlußstutzen erfolgt durch ein Druckstück, das auf dem Katheter gleiten kann und mit einer Schnappverbindung an dem Anschlußstutzen befestigt wird. Im Innern des Druckstilcks befindet sich ein elastomeres Klemmstück, das von dem Ende des Anschlußstutzens im Innern des Druckstücks axial gepreßt wird, so daß es sich radial verformt und den Katheter festklemmt und den Katheterdurchgang abdichtet.

Auch hierbei besteht der Nachteil, daß der Anschlußstutzen eine erhebliche Länge haben muß und daß sein gesamter Umfang für das Aufschieben des Druckstücks verfügbar sein muß. Das Lösen des Katheters von der Kapsel ist nur mit großem Kraftaufwand möglich, wobei das Druckstück zerstört werden kann.

Der Erfindung liegt die Aufgabe zugrunde, eine implantierbare Kathetervorrichtung zu schaffen, die ein sicheres und schnelles Ankuppeln des Katheters an den Anschlußstutzen ermöglicht, bei Bedarf leicht gelöst werden kann und wenig Platz beansprucht, so daß die Abmessungen der Kapsel durch den Anschlußstutzen nicht wesentlich vergrößert werden.

Die Lösung dieser Aufgabe erfolgt erfindungsgemäß mit den Merkmalen des Patentanspruchs 1.

Bei der erfindungsgemäßen implantierbaren Kathetervorrichtung weist der Anschlußstutzen der Kapsel ein Innengewinde auf, in das ein Außengewinde des Druckstücks einschraubbar ist. Da die Außenkontur bzw. die Umfangsfläche des Anschlußstutzens nicht für die Herstellung der Verbindung mit dem Katheter benötigt wird, braucht der Anschlußstutzen keine runde Umfangsfläche aufzuweisen; vielmehr ist der Anschlußstutzen dem Flansch einstückig angeformt und über dem Flansch angeordnet, ohne diesen seitlich (d.h. in Längsrichtung des radial verlaufenden Anschlußstutzens) zu überragen. Die Fixierung und Abdichtung des Katheters in bezug auf den Anschlußstutzen erfolgt ausschließlich im Innern des Anschlußstutzens dadurch, daß das Druckstück in das Innengewinde des Anschlußstutzens eingeschraubt wird und hierbei das Klemmstück axial verformt. Es werden also keine Teile über den Anschlußstutzen gezogen und keine Teile auf dem Umfang des Anschlußstutzens außen befestigt. Die Kapsel hat somit eine geringe radiale Erstreckung.

Der Katheter, der kein speziell geformtes Anschlußstück aufweist, kann vom Anwender auf eine beliebige Länge abgeschnitten werden. Das Katheterende wird lediglich in den Anschlußstutzen der Kapsel eingeführt. Die Fixierung des Katheters erfolgt durch radiales Anpressen des Klemmstücks an den Katheter.

Die Verformung des elastomeren Klemmstücks erfolgt vorzugsweise durch zwei als Innenkonus ausgebildete Stirnflächen, von denen die eine an der Kapsel und die andere am Druckstück angeordnet ist und denen die Außenflächen der Stirnwände des Klemmstückes angepaßt sind. Das Klemmstück ist als rohrförmiger Ring ausgebildet, dessen Stirnwände Öffnungen zum passenden Durchlaß des Katheters aufweisen und bei dem der zylindrische Bereich zwischen den beiden Stirnwänden einen größeren Durchmesser als der Katheter hat, so daß das Klemmstück in diesem Bereich von dem Katheter abgehoben ist. An den konischen Stirnflächen ergibt sich eine verstärkte radiale Anpressung der konischen Stirnwände des Klemmstückes an den Katheter, so daß zwei hintereinander angeordnete Dichtflächen an den beiden Enden des Klemmstückes erzeugt werden. Infolge des von dem Katheter entfernten Zentralbereiches des Klemmstückes hat das Klemmstückmaterial bei axialer Pressung eine gewisse Ausweichmöglichkeit und es wird verhindert, daß der Katheter zusammengequetscht und abgesperrt wird, wenn das Druckstück in einer für eine ausreichende Abdichtung notwendig erscheinenden Weise in den Anschlußstutzen eingeschraubt worden ist.

Im folgenden werden unter Bezugnahme auf die Zeichnungen Ausführungsbeispiele der Erfindung näher erläutert.

Es zeigen:

Fig. 1     einen Längsschnitt durch die Kapsel mit eingeführtem Katheterende bei einem ersten Ausführungsbeispiel,

Fig. 2     eine Draufsicht der Kapsel nach Fig. 1 und

Fig. 3     einen Längsschnitt durch den Anschlußstutzen bei festgeklemmtem Katheter bei einem zweiten Ausführungsbeispiel.

Die Kathetervorrichtung weist eine Kapsel 10 und einen Katheter in Form eines flexiblen Schlauchs auf, dessen Lumen mit einem Hohlraum 12 im Innern der Kapsel 10 verbunden ist. Der Hohlraum 12 dient zur Aufnahme eines flüssigen Medikaments, das durch den Katheter 11 in ein Gefäß eines Patienten eingeleitet werden kann. Die Kapsel 10 weist eine Bodenwand 13, eine Umfangswand 14 und eine perforierbare Oberwand 15 auf. Diese Wände 13, 14 und 15 umschließen den Hohlraum 12. Die Oberwand 15 besteht aus einer Membran, die mit einer (nicht dargestellten) Hohlnadel durchstochen werden kann und die sich nach dem Herausziehen der Hohlnadel wieder abdichtend schließt.

Das Gehäuse der Kapsel 10 besteht aus zwei Gehäuseteilen 16 und 17. Das innere Gehäuseteil 16 bildet die Bodenwand 13 und den unteren Teil der Umfangswand 14, und das äußere Gehäuseteil 17 umgibt das innere Gehäuseteil 16 umfangsmäßig und überragt dieses Gehäuseteil nach oben. Am oberen Ende weist das Gehäuseteil 17 einen ringförmigen wulstartigen Kragen 18 auf, der von oben her gegen den Rand der Oberwand 15 drückt und diesen Rand gegen die obere Stirnseite des inneren Gehäuseteils 16 gedrückt hält. Die Gehäuseteile 16 und 17 sind miteinander verklebt oder verschweißt. Der wulstförmige Kragen 18 bildet eine trichterförmige Begrenzung der Oberwand 15, wodurch das Auffinden der Oberwand und das Durchstechen mit der Hohlnadel erleichtert wird.

Die Kapsel 10 wird unter der Haut des Patienten implantiert, wobei die Oberwand 15 der Haut zugewandt, also nach außen gerichtet ist. Der Rand der Oberwand 15 ist stufenförmig abgesetzt, so daß der obere Teil der Oberwand 15 in den von dem Kragen 18 umschlossenen Bereich hinein nach oben vorsteht.

Das äußere Gehäuseteil 17, das in Draufsicht kreisrund ist, weist an seinem unteren Ende einen radial abstehenden ringförmigen Flansch 19 auf, der mit Löchern 20 zum Fixieren der Kapsel an der Faszie versehen ist. Der Flansch 19 umgibt die Bodenwand 13 des Hohlraums 12 und befindet sich in der gleichen Ebene wie die Bodenwand. Die Gehäuseteile 16 und 17 sind passend so ineinandergesetzt, daß sie spielfrei (ohne Hohlräume) ineinanderpassen und daß die Kapsel 10 eine kontinuierliche ebene Unterseite aufweist.

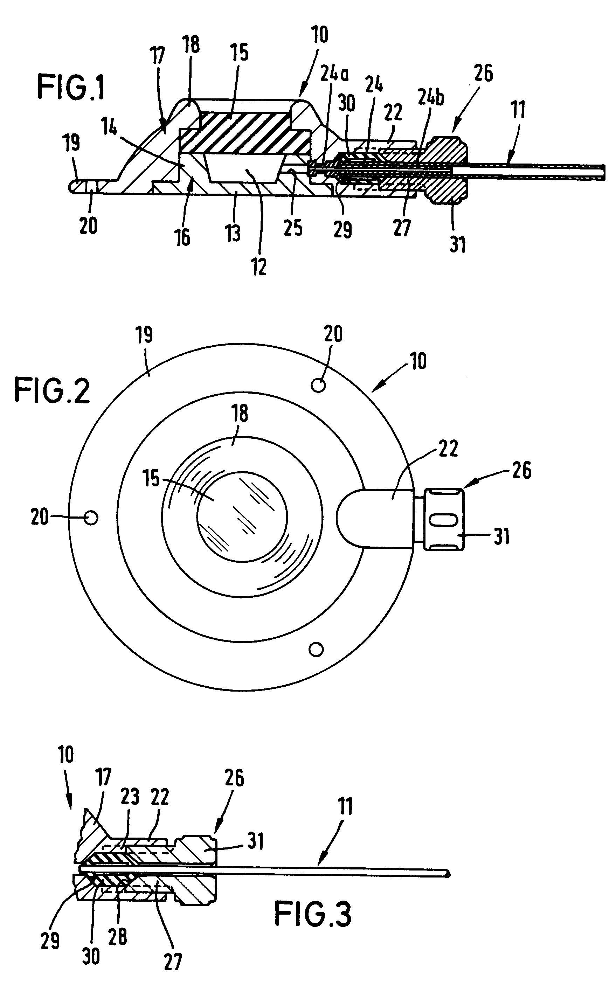

Der Anschlußstutzen 22 ist dem äußeren Gehäuseteil 17 einstückig angeformt. Dieser Anschlußstutzen erhebt sich von dem Flansch 19 nach oben und geht in den Seitenwandbereich des äußeren Gehäuseteils 17 über. Wie insbesondere aus Fig. 2 zu ersehen ist, steht der Anschlußstutzen 22 nicht über den Flansch 19 hinaus seitlich vor, so daß er die radialen Abmessungen der Kapsel nicht vergrößert.

Der Anschlußstutzen 22 weist einen zylindrischen Klemmraum auf, dessen Umfangswand auf einem Teil der Länge von dem Innengewinde 23 gebildet wird. In den Anschlußstutzen 22 führt der Katheter 11 hinein. In das festzulegende Katheterende ragt die Stützkanüle 24 hinein. Diese Stützkanüle weist einen Befestigungsabschnitt 24a auf, der an der Kapsel 10 fixiert, z.B. von dem Kunststoffmaterial des Gehäuseteils 17 umspritzt, ist, und einen geradlinigen rohrförmigen Stützabschnitt 24b, der in dem Anschlußstutzen 22 koaxial verläuft und unabgestützt nach außen ragt. Die Stützkanüle 24 weist einen von einem Ende zum anderen durchgehenden Längskanal auf, der über eine Bohrung 25 des Gehäuseteils 16 mit dem Hohlraum 12 verbunden ist.

Auf den Katheter 11 ist das Druckstück 26 aufgeschoben, das einen Längskanal aufweist, in dem der Katheter 11 leicht verschiebbar ist. Das Druckstück 26 ist mit einem Gewindeschaft 27 versehen, dessen Außengewinde mit dem Innengewinde 23 zusammengreift. Am rückwärtigen Ende des Gewindeschafts 27 befindet sich der Drehkopf 31, der Greifrippen aufweist, um gedreht zu werden. Der Stützabschnitt 24b der Stützkanüle 24 erstreckt sich im Katheter 11 bis in das Druckstück 26 hinein.

In der vorderen Stirnfläche des Gewindeschafts 27 des Druckstücks 26 ist eine trichterförmige Vertiefung 28 vorgesehen, die den Klemmraum begrenzt. Die gegenüberliegende Begrenzung des Klemmraums wird von der trichterförmigen Stirnfläche 29 des Stützflansches der Stützkanüle 24 gebildet. Gegen diese Stirnfläche 29 stößt das Katheterende.

Der Klemmraum wird von dem elastomeren Klemmstück 30, das in Fig. 1 im ungespannten Zustand dargestellt ist, im wesentlichen ausgefüllt. Dieses Klemmstück besteht aus einem rohrförmigen Ring aus elastomerem Material. Durch die zylindrische Mittelöffnung des Ringes, deren Querschnitt größer ist als der Außendurchmesser des Katheters, führt der Katheter 11 hindurch, und die auf der Innenseite zur Klemmstücklängsachse senkrecht verlaufenden Stirnwände des Ringes sind auf der Außenseite in Anpassung an die trichterförmigen Stirnflächeen 28 und 29 kegelstumpfförmig ausgebildet.

Wenn das Druckstück 26 in das Innengewinde 23 eingeschraubt wird, wird das Klemmstück 30 axial (bezogen auf den Katheter 11) zusammengedrückt, wobei es sich gleichzeitig radial aufweitet. Die größte radiale Pressung erfolgt im Bereich der trichterförmigen Stirnflächen 28 und 29 (Fig. 3), so daß das Klemmstück 30 an seinen beiden Enden verstärkt gegen den Umfang des Katheters 11 gedrückt wird, während der Bereich zwischen den Enden weniger stark an den Katheter angedrückt wird oder sogar von diesem abhebt. Auf diese Weise werden an den Enden des Klemmstücks 30 zwei Druck- und Abdichtbereiche geschaffen, durch die der Katheter 11 relativ zur Kapsel 10 fixiert wird. Außerdem erfolgt eine Abdichtung des Katheterdurchgangs durch den Anschlußstutzen 20, so daß keine Flüssigkeit außen am Katheter entlang aus dem Hohlraum 12 ausfließen kann.

Wie aus Fig. 2 erkennbar ist, ragt nur ein Teil des Druckstücks 26 über die Kontur der Kapsel 10 in radialer Richtung hinaus.

Zum Befestigen des Katheters 11 an der Kapsel 10 wird das Druckstück 26 losgeschraubt, so daß das Klemmstück 30 entlastet ist. Der Katheter 11 wird durch das Druckstück 31 hindurch auf den Stützabschnitt 24b der Stützkanüle 24 aufgeschoben, bis das Katheterende gegen die Stirnfläche 29 stößt. Dann wird das Druckstück 26 festgeschraubt, wobei das Klemmstück 30 die in Fig. 1 dargestellte Form annimmt und den Katheter radial gegen den Stützbereich 24b preßt.

Fig. 3 zeigt ein Ausführungsbeispiel, bei dem eine Stützkanüle nicht vorhanden ist. In diesem Fall ist das Klemmstück 30 an der trichterförmigen Stirnfläche 29 des Gehäuseteils 17 abgestützt. Im übrigen entspricht das Ausführungsbeispiel von Fig. 3 demjenigen der Fign. 1 und 2.

## Patentansprüche

1. Implantierbare Kathetervorrichtung mit

einer Kapsel (10), die einen Hohlraum (12), eine Bodenwand (13), eine Umfangswand (14) und eine mit einer Nadel perforierbare Oberwand (15) sowie einen in der Ebene der Bodenwand (13) liegenden Umfangsflansch (19) aufweist,

einem von der Umfangswand (14) der Kapsel (10) seitlich abgehenden Anschlußstutzen (12),

einem Katheter (11), der in den Anschlußstutzen (22) einschiebbar ist,

einem elastomeren Klemmstück (30), das mit dem Anschlußstutzen (22) zusammenwirkt, um den Katheter (11) relativ zu dem Anschlußstutzen (22) zu fixieren und abzudichten,

und einem mit dem Anschlußstutzen (22) verbindbaren Druckstück (26), das den Katheter (11) umgibt und axial derart gegen das Klemmstück (30) drückt, daß dieses radial deformiert wird und den Durchgang des Katheters durch den Anschlußstutzen (22) abdichtet, **dadurch gekennzeichnet,** daß der Anschlußstutzen (22) über dem Umfangsflansch (19) angeordnet ist, ohne diesen seitlich zu überragen, und daß der Anschlußstutzen (22) einen durch eine kapselseitige und durch eine druckstückseitige Stirnfläche (29,28) begrenzten Klemmraum zur Aufnahme des elastomeren Klemmstücks (30) enthält und ein Innengewinde (23) aufweist, in das ein Außengewinde des Druckstücks (26) einschraubbar ist.

2. Kathetervorrichtung nach Anspruch 1, dadurch gekennzeichnet, daß der Anschlußstutzen (22) dem Flansch (19) einstückig angeformt ist.

3. Kathetervorrichtung nach Anspruch 1 oder 2, dadurch gekennzeichnet, daß die kapselseitige Stirnfläche (29) trichterförmig ist.

4. Kathetervorrichtung nach einem der Ansprüche 1 bis 3, dadurch gekennzeichnet, daß die dem Klemmstück (30) zugewandte Stirnfläche (28) des Druckstücks (26) eine trichterförmige Vertiefung aufweist.

5. Kathetervorrichtung nach Anspruch 1, dadurch gekennzeichnet, daß die Kapsel (10) ein erstes Gehäuseteil (16) aufweist, das die Bodenwand (13) und einen unteren Teil der Umfangswand (14) bildet, und ein zweites Gehäuseteil (17), das das erste Gehäuseteil (16) seitlich umgibt und einen Kragen (18) aufweist, welcher über die Oberwand (15) hinaus vorsteht und von oben gegen einen Rand der perforierten Oberwand (15) drückt.

6. Kathetervorrichtung nach Anspruch 5, dadurch gekennzeichnet, daß der Anschlußstutzen (22) und der Flansch (19) Bestandteile des zweiten Gehäuseteils (17) sind.

**Claims**

1. Implantable catheter assembly comprising

   - a capsule (10) including a cavity (12), a bottom wall (13), a peripheral wall (14), a top wall (15) pierceable by a needle and a peripheral flange (19) disposed in the plane of said bottom wall (13),
   - a connecting stub (22) projecting laterally from said peripheral wall (14) of said capsule (10),
   - a catheter (11) insertable into said connecting stub (22),
   - an elastomeric clamping member (30) coacting with said connecting stub (22) to fix and seal said catheter (11) relative to said connecting stub (22),
   - and a pressure member (26) adapted to be joined to said connecting stub (22) and enclosing said catheter (11) to press it axially against said clamping member (30) so that the latter is deformed radially and seals the passage of said catheter through said connecting stub (22), **characterized in that**
   - said connecting stub (22) is arranged above said flange (19) without projecting beyond it laterally. and that said connecting stub (22) includes a retaining chamber for receiving said elastomeric clamping member (30), said retaining chamber being defined by end walls (29, 28) facing said capsula and said pressure member, respectively, and having an internal thread (23) into which an external thread of said pressure member (26) can be screwed.

2. Catheter means according to claim 1, characterized in that said connecting stub (22) is integrally formed with said flange (19).

3. Catheter means according to claim 1 or 2, characterized in that said end wall (29) facing said capsula is funnel-shaped.

4. Catheter means according to one of claims 1 to 3, characterized in that said end wall (28) of said pressure member (26) facing said clamping member (30) is provided with a funnel-shaped recess.

5. Catheter means according to claim 1, characterized in that said capsule (10) contains a first housing part (16) forming said bottom wall (13) and a lower portion of said peripheral wall (14), and a second housing part (17) laterally enclosing said first housing part (16) and comprising a collar (18) projecting above said top wall (15) and pressing from above against an edge of said pierced top wall (15).

6. Catheter means according to claim 5, characterized in that said connecting stub (22) and said flange (19) form part of said second housing part (17).

**Revendications**

1. Dispositif de cathéter implantable comportant

   une capsule (10), qui présente une cavité (12), une paroi de fond (13), une paroi circonférentielle (14) et une paroi supérieure (15) pouvant être perforée par une aiguille, ainsi qu'une bride circonférentielle (19) située dans le plan de la paroi de fond (13),

   une tubulure de raccordement (22) qui s'étend latéralement à partir de la paroi circonférentielle (14) de la capsule (10),

   un cathéter (11), qui peut être inséré dans la tubulure de raccordement (22),

   une pièce élastomère de serrage (30), qui coopère avec la tubulure de raccordement (22) pour fixer et étanchéifier le cathéter (11) par rapport à la tubulure de raccordement (22), et

   une pièce de pression (26), qui peut être raccordé à la tubulure de raccordement (22), entoure le cathéter (11) et le presse axialement contre la pièce de serrage (30) de telle sorte que cette dernière est déformée radialement et étanchéifie le passage du cathéter à travers la tubulure de raccordement (22),

   caractérisé en ce

   que la tubulure de raccordement (22) est disposée au-dessus de la bride circonférentielle (19), sans faire saillie latéralement au-delà de cette dernière et que la tubulure de raccordement (22) présente un espace de serrage, qui est limité par une surface frontale (29) située du côté de la capsule et par une surface frontale (28) située du côté de la pièce de pression, pour la réception de la pièce élastomère de serrage (30) et un taraudage (23), dans lequel peut être vissé un filetage extérieur de la pièce de pression (26).

2. Dispositif de cathéter selon la revendication 1, caractérisé en ce que la tubulure de raccordement (22) est formée par moulage d'un seul tenant sur la bride (19).

3. Dispositif de cathéter selon la revendication 1 ou 2, caractérisé en ce que la face frontale (29) située du côté de la capsule possède une forme d'entonnoir.

4. Dispositif de cathéter selon l'une des revendications 1 à 3, caractérisé en ce que la surface frontale (28) de la pièce de pression (26), orientée vers la pièce de serrage (30), présente un renfoncement en forme d'entonnoir.

5. Dispositif de cathéter selon la revendication 1, caractérisé en ce que la capsule (10) présente une première partie de boîtier (16), qui forme la paroi de fond (13) et une partie inférieure de la paroi circonférentielle (14), et une seconde partie de boîtier (17), qui entoure latéralement la première partie de boîtier (16) et présente un collet (18), qui fait saillie at-dessus de la paroi supérieure (15) et presse à partir du haut contre un bord de la paroi supérieure perforée (15).

6. Dispositif de cathéter selon la revendication 5, caractérisé en ce que la tubulure de raccordement (22) et la bride (19) sont des parties constitutives de la seconde partie de boîtier (17).

FIG.1

FIG.2

FIG.3